(19) Europäisches Patentamt
European Patent Office
Office européen des brevets

(11) Veröffentlichungsnummer : **0 030 676**
**B1**

(12) **EUROPÄISCHE PATENTSCHRIFT**

(45) Veröffentlichungstag der Patentschrift : **08.02.84**

(21) Anmeldenummer : **80107536.7**

(22) Anmeldetag : **03.12.80**

(51) Int. Cl.$^3$ : **C 07 C103/76**, C 07 C 93/233, C 07 C 79/46, C 07 D211/16, C 07 D231/12, A 01 N 37/48, A 01 N 43/40, A 01 N 43/48

(54) **Phenoxybenzoesäure-Derivate, Verfahren zu ihrer Herstellung sowie ihre Verwendung als Herbizide und Pflanzenwuchsregulatoren.**

(30) Priorität : **14.12.79 DE 2950401**

(43) Veröffentlichungstag der Anmeldung : **24.06.81 Patentblatt 81/25**

(45) Bekanntmachung des Hinweises auf die Patenterteilung : **08.02.84 Patentblatt 84/06**

(84) Benannte Vertragsstaaten : **AT BE CH DE FR GB IT LI NL**

(56) Entgegenhaltungen :
**EP-A- 0 003 584**
**EP-A- 0 013 660**
**EP-A- 0 014 900**
**EP-A- 0 020 052**
**EP-A- 0 027 837**
**DE-A- 2 720 427**
**DE-A- 2 753 900**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Förster, Heinz, Dr.**
**Am Eckbusch 47**
**D-5600 Wuppertal 1 (DE)**
Erfinder : **Eue, Ludwig, Dr.**
**Paul-Klee-Strasse 36**
**D-5090 Leverkusen (DE)**
Erfinder : **Schmidt, Robert, Rudolf, Dr.**
**Hahnenweg 5**
**D-5000 Köln 80 (DE)**
Erfinder : **Lürssen, Klaus, Dr.**
**August-Kierspel-Strasse 89**
**D-5060 Bergisch Gladbach 2 (DE)**

**EP 0 030 676 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Phenoxybenzoesäure-Derivate, Verfahren zu ihrer Herstellung sowie ihre Verwendung als Herbizide und Pflanzenwuchsregulatoren

Die Erfindung betrifft neue Phenoxybenzoesäure-Derivate, ein Verfahren zu ihrer Herstellung sowie ihre Verwendung als Herbizide und Pflanzenwuchsregulatoren.

Es ist bereits bekannt geworden, daß bestimmte Phenoxybenzoesäure-Derivate herbizide Eigenschaften besitzen (vergleiche DE-OS 2 311 638 und US-PS 3 928 416). Die Wirkung dieser Stoffe ist bei einem Einsatz nach dem Pre-emergence-Verfahren gut. Nachteilig ist jedoch, daß einige Problemunkräuter und Ungräser nicht immer voll bekämpft werden.

Weiterhin wurden in den vorgängigen Europäischen Offenlegunsschriften 20 052 und 27 837 Diphenylether mit herbiziden Eigenschaften offenbart. In der EP-A 20 052 werden allerdings keine Verbindungen beschrieben, in denen die zur Nitro-Gruppe ortho-Ständige Seitenkette einen über Stickstoff gebundenen Stickstoff-Heterocyclus enthält. Im Falle der in der EP-A 27 837 aufgeführten Diphenylether kann die zur Nitro-Gruppe benachbarte Seitenkette zwar einen über Carbonyl gebundenen gesättigen Stickstoff enthaltenden heterocyclischen Ring bedeuten, die entsprechenden ungesättigten Verbindungen werden jedoch nicht erwähnt.

Aus der DE-A 27 20 427 sind ebenfalls zahlreiche herbizid wirksame Diphenylether bekannt. Es werden aber keine Verbindungen offenbart, in dene die beiden para-Positionen des Diphenylethers durch einen Trifluormethyl-Rest und eine Nitro-Gruppe besetzt sind.

Schießlich werden in der DE-A 27 53 900 herbizid wirksame Phenoxybenzoesäure-Derivate beschrieben, in denen der über die Carbonylgruppe der Benzoesäure gebundene Rest auch NRZ' bedeuten kann, wobei Z' außer für eine Epoxyalkyl-Gruppe ausschließlich für offenkettige, ungesättigte Reste steht. Amide, die an der entsprechenden Stelle des Moleküls jeweils einen stickstoffhaltigen Heterocyclus oder eine Esteramid-Einheit aufweisen, werden dort nicht erwähnt.

Es wurden nun neue Phenoxybenzoesäure-Derivate der Formel

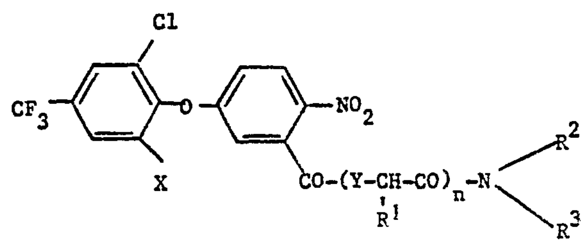

(I)

in welcher

$R^1$ für Wasserstoff oder Methyl steht,

n für 0 oder 1 steht,

X für Wasserstoff oder Chlor steht,

Y für Sauerstoff oder Imino (NH) steht und

$R^2$ und $R^3$ zusammen mit dem Stickstoffatom, an das sie gebunden sind, für Monoalkyl-piperidyl mit 1 bis 3 Kohlenstoffatomen in der Alkylgruppe stehen, sofern n für 1 steht, und außerdem

$R^2$ und $R^3$ zusammen mit dem Stickstoffatom, an das sie gebunden sind, für gegebenenfalls durch $C_1$-$C_3$-Alkyl substituiertes Pyrazolyl stehen, sofern n für 0 seht, gefunden.

Weiterhin wurde gefunden, daß man die neuen Phenoxybenzoesäure-Derivate der Formel (I) erhält, wenn man Phenoxybenzoesäure-chloride der Formel

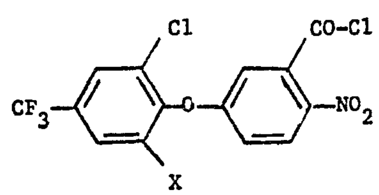

(II)

in welcher

X die oben angegebene Bedeutung hat, mit Verbindungen der Formel

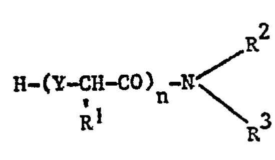

(III)

in welcher

$R^1$, $R^2$, $R^3$, Y und n die oben angegebene Bedeutung haben, gegebenenfalls in Gegenwart eines Säureakzeptors und gegebenenfalls unter Verwendung eines Verdünnungsmittels umsetzt.

Schließblich wurde gefunden, daß sich die neuen Phenoxy-benzoesäure-Derivate der Formel (I) durch eine hervorragende herbizide und pflanzenwuchsregulierende Wirksamkeit auszeichnen. Die erfindungsgemäßen Wirsktoffe können insbesondere im Pre-emergence-Verfahren zur Bekämpfung von Umkräutern und Ungräsern in wichtigen Kulturen, wie z. b. in Getreide und in Sojabohnen, eingesetzt werden. Die pflanzenwuchsregulierende Wirkung der neuen Verbindungen kann insbesondere in der Anwendung als Baumwolldefoliants genutzt werden.

Überraschenderweise zeigen die erfindungsgemäßen Phenoxybenzoesäure-Derivate der Formel (I) eine wesentlich bessere herbizide und pflanzenwuchsregulierende Wirksamkeit als aus dem Stand der Technik bekannte Verbindungen analoger Struktur und gleicher Wirkungsrichtung.

So übertreffen die erfindungsgemäßen Stoffe selbst die 3-(2-Chlor-4-trifluormethylphenoxy)-6-nitro-benzoesäure bezüglich ihrer herbiziden Eigenschaften. Eine derartige Überlegenheit ist völlig überraschend, denn bei der genannten Substanz handelt es sich nicht nur um eine Verbindung, die den erfingungsgemäßen Stoffen konstitutionell ähnlich ist, sondern auch um die aktive Komponente eines im Handel befindlichen bewährten Herbizids.

Im übrigen konnte die hervorragende herbizide Wirksamkeit der erfindungsgemäßen Stoffe auch unter Berücksichtigung der technischen Lehre, die aus der DE-A 27 20 427 und der DE-A 27 53 900 hervorgeht, nicht erwartet werden, denn die im vorliegenden Fall beanspruchten Phenoxybenzoesäure-Derivate unterscheiden sich von den aus diesen Offenlegungsschriften bekannten Verbindungen in konstitutionell signifikanter Weise.

Verwendet man bei dem erfindungsgemäßen Verfahren 5-(2,6-Dichlor-4-trifluormethyl-phenoxy)-2-nitro-benzoesäure-chlorid und Hydroxyessigsäure-2-methyl-piperidid als Ausgangsstoffe, so kann der Verlauf der Reaktion durch das folgende Formelschema wiedergegeben werden :

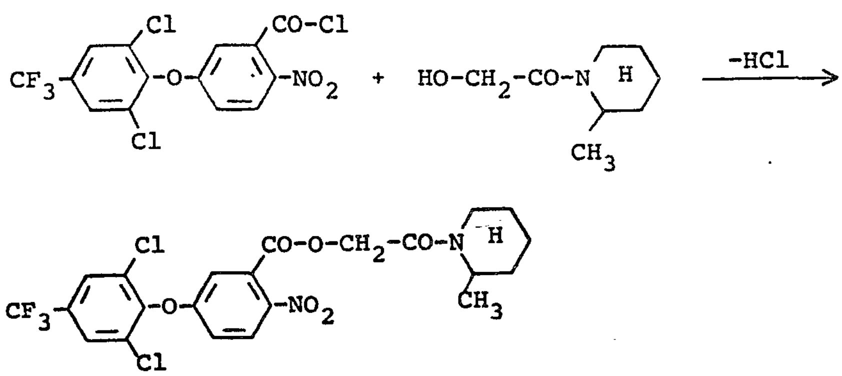


Die bei dem erfindungsgemäßen Verfahren als Ausgangsstoffe benötigten Phenoxybenzoesäurechloride sind durch die Formel (II) eindeutig definiert. In dieser Formel steht X für Wasserstoff oder Chlor.

Als Beispiele für die Phenoxybenzoesäurechloride der Formel (II) seien 5-(2-Chlor-4-trifluormethyl-phenoxy)-2-nitro-benzoesäurechlorid und 5-(2,6-Dichlor-4-trifluormethyl-phenoxy)-2-nitro-benzoesäurechlorid genannt.

Die Phenoxybenzoesäurechloride der Formel (II) sind in der Literatur bisher noch nicht beschrieben worden. Man erhält sie jedoch auf einfache Weise, indem man Phenoxybenzoesäuren der Formel

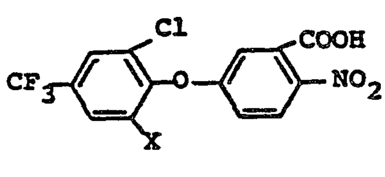

(IV)

in welcher

X für Wasserstoff oder Chlor steht, mit Chlorierungsmitteln, wie z. B. Thionylchlorid, gegebenenfalls unter Verwendung eines Katalysators, wie z. B. Dimethylformamid, und gegebenenfalls unter Verwendung eines Verdünnungsmittels, wie z. B. 1,2-Dichlorethan, bei Temperaturen zwischen 10 und 100 °C, umsetzt und anschließend flüchtige Komponenten unter vermindertem Druck abdestilliert.

Die Phenoxybenzoesäuren der Formel (IV), — nämlich 5-(2-Chlor-4-trifluormethyl-phenoxy)-2-nitro-benzoesäure und 5-(2,6-dichlor-4-trifluormethylphenoxy)-2-nitro-benzoesäure — , sind bereits bekannt (vergleiche US-PS 3 928 416).

Die beim erfindungsgemäßen Herstellungsverfahren weiterhin als Ausgangsstoffe benötigten Verbindungen sind durch die Formel (II) definiert. Als Beispiele für Stoffe der Formel (III) seien genannt :

Hydroxyessigsäure-pyrazolid-3,5-dimethyl-pyrazolid-2-methyl-, -3-methyl-, -4-methyl-, -2-ethyl- und -4-ethyl-piperidid.

Die Verbindungen der Formel (III), in welchen n für Null steht, sind bekannt. Diejenigen Stoffe der Formel (III), in denen n für 1 steht, sind ebenfalls bekannt oder lassen sich nach üblichen Verfahren herstellen. So erhält man die α-Hydroxycarbonsäure-amide der Formel

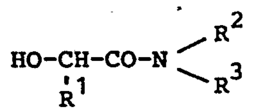

(IIIa)

in welcher

$R^1$, $R^2$ und $R^3$ die oben angegebene Bedeutung haben, indem man α-Halogen-carbonsäureamide der Formel

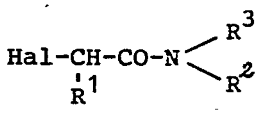

(V)

in welcher

$R^1$, $R^2$ und $R^3$ die oben angegebene Bedeutung haben und

Hal für Chlor oder Brom steht, in einer ersten Stufe mit überschüssigem Natrium- oder Kalium-acetat, gegebenenfalls in Gegenwart eines Katalysators, wie z. B. Tetrabutylammoniumbromid, und gegebenenfalls unter Verwendung eines Verdünnungsmittels, wie z. B. Essigsäure oder Toluol, bei Temperaturen zwischen 20 und 200° C umsetzt und in einer zweiten Stufe die dabei entstehenden Acetoxycarbonsäureamide der Formel

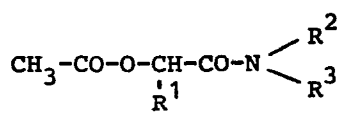

(VI)

in welcher

$R^1$, $R^2$ und $R^3$ die oben angegebenen Bedeutungen haben, durch Umsetzung mit verdünnter wässrig-alkoholischer Natronlauge bei Temperaturen zwischen 20 und 150° C entacyliert (vergleiche DE-OS 2 201 432 und US-PS 3 399 988).

Die α-Halogencarbonsäureamide der Formel (V) sind bekannt oder können analog bekannten Verfahren hergestellt werden. Man erhält sie z. B. durch Umsetzung von α-Halogen-carbonsäurehalogeniden, wie z. B. Chloracetylchlorid, mit der jeweils benötigen heterocyclischer Verbindung gegebenenfalls in Gegenwart eines Säureakzeptors, wie z. B. Kaliumhydroxid (verglieche J. Agric. Food Chem. *4* (1956), 518-522).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethyl-ether und Diglycoldimethylether, Tetrahydrofuran und Dioxan, Ketone, wie Aceton, Methyl-ethyl-, Methyl-isopropyl-, und Methyl-isobutyl-keton, Ester, wie Essigsäure-methylester und -ethylester, Nitrile, wie z. B. Acetonitril und Propionitril, Amide, wie z. B. Dimethylformamid, Dimethylacetamid und N-Methyl-pyrrolidon, sowie Dimethylsufloxid, Tetramethylensulfon und Hexamethylphosphorsäuretriamid.

Als Säureakzeptoren können alle üblichen Säurebindemittel Verwendung finden. Besonders bewährt haben sich Alkali-hydroxide, wie z. B. Natrium- und Kaliumhydroxid, Alkalicarbonate und -alkoholate, wie Natrium- und Kaliumcarbonat, Natrium- und Kaliumethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen – 20 und 100 °C, vorzugsweise zwischen 0 bis 50 °C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in angenähert äquimolaren Mengen eingezetzt. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperature gerührt. Danach gibt man ein organisches Lösungsmittel, z. B. Toluol, zu und arbeitet die organische Phase wie üblich durch Waschen, Trocknen und Abdestillieren des Lösungsmittels auf.

Die neuen Verbindungen fallen zum Teil in Form von Ölen an, die sich zum Teil nicht unzersetzt destillieren lassen, jedoch durch sogenanntes « Andestillieren », d. h. durch längeres Erhitzen unter

vermindertem Druck auf mäßig erhöhte Temperaturen von den letzten flüchtigen Anteilen befreit und auf diese Weise gereinigt werden. Zu ihrer Charakterisierung dient der Brechungsindex. Soweit die neuen Produkte in fester Form anfallen, können sie durch Umkristallisation gereinigt werden. Zur Charakterisierung dient dann der Schmelzpunkt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut in weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthis, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hofenanlagen und zur selektiven Unkrautbekämpfung in einjährigen Kulturen eingesetzt Werden.

Die erfindungsgemäßen Wirkstoffe besitzen außerdem eine sehr gute pflanzenwuchsregulierende Wirksamkeit. Sie eignen sich besonders gut zur Wuchshemmung, sowie zur Entlaubung und Austrocknung der Blätter bei Baumwolle und Kartoffeln. Die Wirkstoffe können in die üblichen Formulierung übergeführt werden, wie Lösungen, Emulsionen, Spritzpulver, Suspensionen, Pulver, Stäubemittel, lösliche Pulver, Granulate, Suspensions-Emulsionskonzentrate, Wirsktoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln, und/oder festen Trägerstoffen, gegebenenfalls unter Verwendung von oberflächenaktiven Mittlen, also Emulgiermitteln und/oder Dispergiermitteln und/oder Schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage :

Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z. B. Erdölfraktionen, Alkohole, wie Butanol oder Glycol sowie deren Ester, Ketone, wie Aceton, Methylethylketon, Methylisobutylketon oder Cyclohexanon, starke polare Lösungsmittel, wie Dimethylformamid und Dimethylsulfoxid, sowie Wasser.

Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Caclit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate ; als Dispergiermittel kommen in Frage : z. B. Lingin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkkohol, Polyvinylacetat.

Es können Farbstoffe wie anorganische Pigmente, z. B. Eisenoxid, Titanoxid, Ferrocyanblau und

organische Farbstoffe, wie Alizarin-, Azofarbstoffe, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

## Herstellungsbeispiele

### Beispiel 1

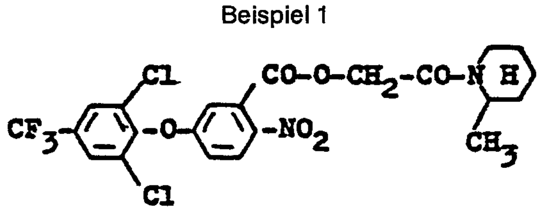

6,6 g Hydroxyessigsäure-2-methylpiperidid werden zusammen mit 8 ml Pyridin in 60 ml Toluol vorgelegt. Bei 0 °C wird 2-Nitro-5-(2,6-Dichlor-4-trifluormethyl-phenoxy)-benzoesäure-chlorid, gelöst in 30 ml Toluol, zugetropft. Dann wird 12 Stunden bei 20 °C gerührt. Dann werden 100 ml Toluol zugegeben und die organische Phase wird alkalisch und mit Wasser neutral gewaschen. Das Toluol wird abdestilliert. Zürück bleiben 14,8 g (70 % der Theorie) 5-(2,6-Dichlor-4-trifluormethyl-phenoxy)-2-nitro-benzoesäure-(2-methyl-piperidyl-carbonyl-methyl)-ester mit dem Schmelzpunkt 50 °C.

### Beispiel 2

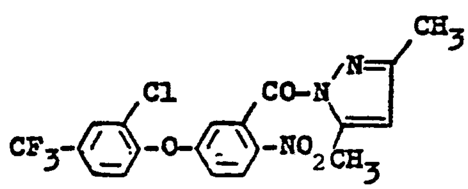

6 g 3,5-Dimethylpyrazol, 6,3 g Triethylamin und 90 ml Toluol weden vorgelegt. Bei 0-5 °C werden 25 g 2-Nitro-5-(2-Chlor-4-trifluormethyl-phenoxy) benzoesäurechlorid, gelöst in 50 ml Toluol, zugetroft. Es wird 12 Stunden bei 20 °C gerührt. Dann werden 100 ml Toluol zugesetzt und die organische Phase wird je 1 mal sauer, alkalisch und neutral gewaschen. Das Toluol wird abdestilliert. Zurück bleibt ein langsam kristallisierendes Öl. Man erhält 25 g (88 % der Theorie) 5-(2-Chlor-4-trifluormethyl-phenoxy)-2-nitro-benzoesäure-3,5-di-methyl-pyrazolid mit dem Schmelzpunkt 123-124 °C.

Analog einem der Beispiele 1 bis 2 können die folgenden Verbindungen hergestellt werden :

### Beispiel 3

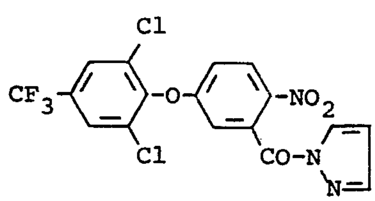

Ausbeute : 87 % der Theorie
Schmelzpunkt : 136 bis 137 °C.

Beispiel 4

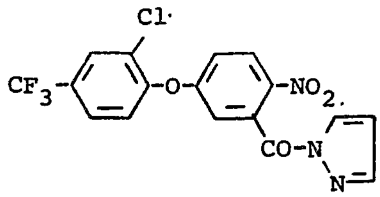

Ausbeute : 77 % der Theorie
Brechungsindex : $n_D^{20}$ = 1,505 7

Beispiel A

Pre-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Zu diesem Zest zeigen die erfindungsgemäßen Wirkstoffe (1) bis (4) eine sehr gute herbizide Wirksamkeit.

Beispiel B

Post-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

In diesem Test zeigen die erfindungsgemäßen Wirkstoffe (1) bis (4) eine sehr gute herbizide Wirksamkeit.

Beispiel C

Wuchshemmung, sowie Entlaubung und Austrocknung der Blätter bei Baumwolle

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gew.-Teil Wirkstoff mit den angegebenen Mengen Lösungsmittel und Emulgator unf füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitung besprüht. Nach 1 Woche werden der Blattfall und das Austrocknen der Blätter im Vergleich zu den Kontrollpflanzen bonitiert. Es bedeuten :

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Text zeigen die erfindungsgemäßen Wirkstoffe (2) und (4) eine starke Wirksamkeit.

**Ansprüche**

1. Phenoxybenzoesäure-Derivate der Formel

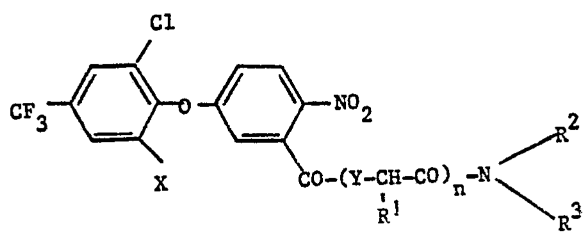

(I)

in welcher

$R^1$ für Wasserstoff oder Methyl steht,
n für 0 oder 1 steht,
X für Wasserstoff oder Chlor steht,
Y für Sauerstoff oder Imino (NH) steht und
$R^2$ und $R^3$ zusammen mit dem Stickstoffatom, an das sie gebunden sind, für Monoalkylpiperidyl mit 1 bis 3 Kohlenstoffatomen in der Alkylgruppe stehen, sofern n für 1 steht, und außerdem
$R^1$ und $R^3$ zusammen mit dem Stickstoffatom, an das sie gebunden sind, für gegebenenfalls durch $C_1$-$C_3$-Alkyl substituiertes Pyrazolyl stehen, sofern n für 0 steht.

2. Verfahren zur Herstellung von Phenoxybenzoesäure-Derivaten der Formel (I), dadurch gekennzeichnet, daß man Phenoxybenzoesäurechloride der Formel

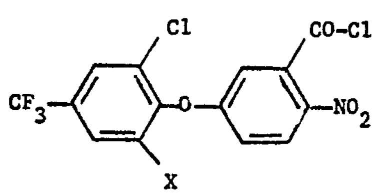

(II)

in welcher

X die oben angegebene Bedeutung hat, mit Verbindungen der Formel

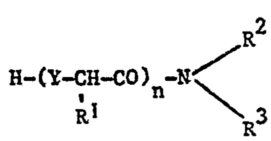

(III)

in welcher

$R^1$, $R^2$, $R^3$, Y und n die oben angegebene Bedeutung haben, gegebenenfalls in Gegenwart eines

Säureakzeptors und gegebenenfalls unter Verwendung eines Verdünnungsmittels umsetzt.

3. Herbizide und pflanzenwuchsregulierende Mittel, gekennzeichnet durch einen Gehalt an mindestens einem Phenoxybenzoesäure-Derivat der Formel (I) gemäß Anspruch 1.

4. Verfahren zur Bekämpfung von Unkräutern sowie zur Regulierung des Pflanzenwachstums, dadurch gekennzeichnet, daß man Phenoxybenzoesäure-Derivate der Formel (I) gemäß Anspruch 1 auf Unkräuter bzw. die zu regulierenden Pflanzen und/oder deren Lebensraum ausbringt.

5. Verwendung von Phenoxybenzoesäure-Derivaten der Formel (I) gemäß Anspruch 1 zur Bekämpfung von Unkräutern sowie zur Regulierung des Pflanzenwachstums.

6. Verfahren zur Herstellung von herbiziden bzw. pflanzenwuchsregulierenden Mitteln, dadurch gekennzeichnet, daß man Phenoxybenzoesäure-Derivate der Formel (I) gemäß Anspruch 1 mit Streckmitteln und/oder oberflächenaktiven Stoffen vermischt.

**Claims**

1. Phenoxybenzoic acid derivatives of the formula

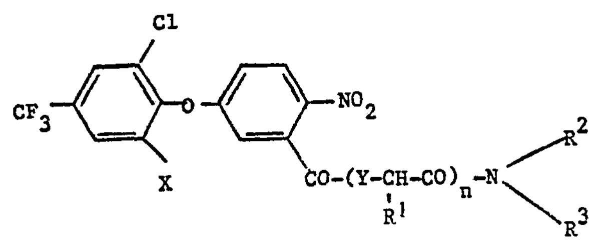

(I)

in which

$R^1$ represents hydrogen or methyl,

n represents 0 or 1,

X represents hydrogen or chlorine,

Y represents oxygen or imino (NH) and

$R^2$ and $R^3$, together with the nitrogen atom to which they are bonded, represent monoalkyl-piperidyl with 1 to 3 carbon atoms in the alkyl group, if n represents 1, and furthermore

$R^2$ and $R^3$, together with the nitrogen atom to which they are bonded, represent pyrazolyl which is optionally substituted by $C_1$-$C_3$-alkyl, if n represents 0.

2. Process for the preparation of phenoxybenzoic acid derivatives of the formula (I), characterised in that phenoxybenzoic acid chlorides of the formula

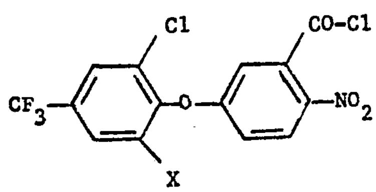

(II)

in which

X has the meaning indicated above, are reacted with compounds of the formula

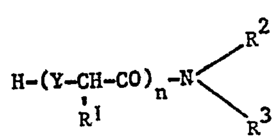

(III)

in which

$R^1$, $R^2$, $R^3$, Y and n have the meaning indicated above, if appropriate in the presence of an acid acceptor and if appropriate using a diluent.

3. Herbicidal and plant growth-regulating agents, characterised in that they contain at least one phenoxybenzoic acid derivative of the formula (I) according to Claim 1.

4. Process for combating weeds and for regulating plant growth, characterised in that phenoxybenzoic acid derivatives of the formula (I) according to Claim 1 are applied to weeds or the plants to be regulated and/or to their environment.

5. Use of phenoxybenzoic acid derivatives of the formula (I) according to Claim 1, for combating weeds and for regulating plant growth.

6. Process for the preparation of herbicidal and plant growth-regulating agents, characterised in that

phenoxybenzoic acid derivatives of the formula (I) according to Claim 1 are mixed with extenders and/or surface-active substances.

**Revendications**

1. Dérivés de l'acide phénoxybenzoïque de formule

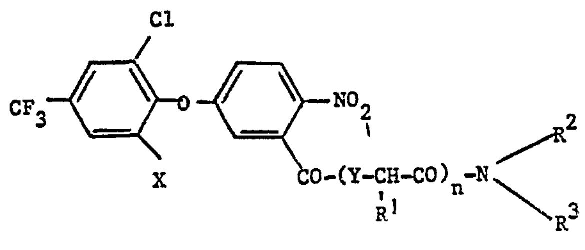

(I)

dans laquelle

$R^1$ représente l'hydrogène ou un groupe méthyle,

n est égal à 0 ou 1,

X représente l'hydrogène ou le chlore,

Y représente l'oxygène ou le groupe imino (NH) et

$R^2$ et $R^3$ forment ensemble et avec l'atome d'azote sur lequel ils sont fixés, un groupe monoalkyl-pipéridyle contenant 1 à 3 atomes de carbone dans la partie alkyle lorsque n est égal à 1, et

$R^2$ et $R^3$ forment ensemble et avec l'atome d'azote sur lequel ils sont fixés un reste pyrazolyle éventuellement substitué par un groupe alkyle en $C_1$-$C_3$ lorsque n est égal à 0.

2. Procédé de préparation des dérivés de l'acide phénoxybenzoïque de formule (1), caractérisé en ce que l'on fait réagir des chlorures d'acides phénoxybenzoïques de formule

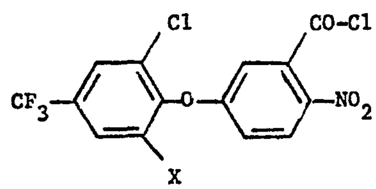

(II)

dans laquelle

X a la signification indiquée ci-dessus, avec des composés de formule

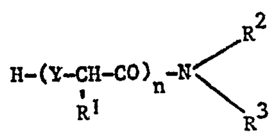

(III)

dans laquelle

$R^1$, $R^2$, $R^3$, Y et n ont les significations indiquées ci-dessus, éventuellement en présence d'un accepteur d'acides et éventuellement en utilisant un diluant.

3. Produits herbicides et régulateurs de la croissance des végétaux, caractérisés en ce qu'ils contiennent au moins un dérivé de l'acide phénoxybenzoïque de formule (I) selon la revendication 1.

4. Procédé pour combattre les mauvaises herbes et pour régler la croissance des végétaux, caractérisé en ce que l'on applique des dérivés de l'acide phénoxybenzoïque de formule (I) selon la revendication 1 sur les mauvaises herbes ou les végétaux dont on veut régler la croissance et/ou sur leur habitat.

5. Utilisation des dérivés de l'acide phénoxybenzoïque de formule (I) selon la revendication 1 pour la lutte contre les mauvaises herbes et la régulation de la croissance des végétaux.

6. Procédé de préparation de produits herbicides et régulateurs de la croissance des végétaux, caractérisé en ce que l'on mélange des dérivés de l'acide phénoxybenzoïque de formule (I) selon la revendication 1 avec des diluants et/ou des substances tensioactives.